# EUROPEAN PATENT APPLICATION

(11) **EP 4 821 535 A2**
(43) Date of publication of application: **14.10.2026**
(21) Application number: 26200697.6
(22) Date of filing: 15.04.2019
(51) Int. Cl.: A61M 25/00

(54) **CATHETER ASSEMBLIES AND RELATED METHODS**

(30) Priority: 18.04.2018 US 201862659332 P
(62) Document number(s) of the earlier application(s) in accordance with Art. 76 EPC: 19720075.1 / 3 781 245
(71) Applicant: B. Braun Melsungen AG, 34212 Melsungen (DE)
(72) Inventor: CHONG, Meng Mun, 10810 Penang (MY); NG, Jarryd Keng Gene, 10810 Penang (MY)
(74) Representative: Bird & Bird LLP

(57) **Abstract**

An intravenous catheter device or apparatus (100) includes a catheter hub (110), a catheter tube (150) that resists kinking, a needle (101), and a needle hub (103). The catheter tube (150) includes a catheter body (151) having a lumen (156), an outer circumference, and a wall thickness between the lumen and the outer circumference, or between an exterior surface (158) and an interior surface (137). The catheter body (151) can have at least two different portions made from two different materials having two different stiffness properties. The first portion (152) can be made from a first material and the second portion (155) can be made from a second material and wherein the stiffness of the second material can be greater than the stiffness of the first material.

## Description

### FIELD OF ART

The present disclosure is generally related to intravenous catheter devices, apparatuses, and assemblies (IVCs) including peripheral and central venous catheter assemblies and more particularly to IVCs with catheter tubes each featuring a stiffened region to help the catheter tube resist kinking while maintaining flexibility, and related methods.

### BACKGROUND

IVCs are common medical invasive devices routinely used for a variety of infusion therapies, including infusing a patient with fluids, withdrawing blood from a patient, or monitoring various parameters of the patient's vascular system. Access to the patient's vasculature is typically accomplished by insertion of a catheter tube known as venipuncture. The catheter tube of the IV catheter assemblies are inserted in a majority of all hospitalized patients during their hospital stay and frequently initiated in many emergency situations.

The insertion procedure for an IVC contains four basic steps: (1) the healthcare worker inserts the needle and the catheter tube together into the patient's vein; (2) after insertion into the vein with the needle point, the catheter tube is forwarded into the vein of the patient by the healthcare worker pushing the catheter tube with his or her finger; (3) the healthcare worker withdraws the needle by grasping the hub end (opposite the point end) while at the same time applying pressure to the patient's skin at the insertion site with his or her free hand to slow down or stop the flow of blood through the catheter tube; and (4) the healthcare worker then tapes the exposed end of the catheter tube and/or the catheter hub to the patient's skin and connects it to the source of the fluid to be administered into the patient's vein.

Because a portion of the catheter tube remains inside the patient, the comfort and safety of the patient can be affected by the flexibility, size (e.g., diameter), and choice of material of the catheter tube. In circumstances where an IVC with a longer catheter tube is needed, the extra length for a given diameter would require a catheter tube with a larger diameter or a stiffer catheter tube to prevent the catheter tube from kinking as the catheter tube is advanced deeper into the vein after venipuncture. A larger diameter catheter tube would require a larger opening at the insertion site and thus a larger needle, which can cause additional pain and discomfort associated with using a larger needle. Additionally, the larger opening at the insertion site increases the risk of infection and recovery time to close the wound. A larger diameter catheter tube may also occlude a larger portion of the inside diameter of the vein. An increased stiffness of the catheter tube can potentially cause injury to the venous valve and the wall of the vein after venipuncture while feeding the catheter tube into the desired location. Additionally, the stiffer catheter tube can cause added discomfort and pain at the insertion site, which could introduce further complications to the patient and delay recovery.

### SUMMARY

The various aspects of an intravenous catheter assembly and catheter tube have several features, no single one of which is solely responsible for their desirable attributes. Without limiting the scope of the present embodiments as set forth in the claims that follow, their more prominent features now will be discussed briefly.

Aspects of the present disclosure include an intravenous catheter assembly comprising at least a catheter tube having at least one spine and related methods for forming the intravenous catheter assembly. The intravenous catheter assembly can be a component or subassembly of a needle device or over-the-needle catheter assembly.

The catheter tubes described herein are usable with catheter hubs described herein and can form part of catheter assemblies described herein.

The spine may be understood as a stiffener as the inclusion of a spine stiffens the portion of the tube body to prevent or limit kinking to the catheter tubing. The spine can have a strip having a surface, a cross-sectional profile having a regular area or an irregular area, such as an oval shape, a square shape, a round shape, a rhombus shape, a polygonal shape, or an irregular shape. The spine can have a length that can extend the full length of the catheter tube or short of the full length of the catheter tube, such as being slightly recessed from the distal opening of the catheter tube.

The catheter tube can include a catheter body comprised of a first flexible portion and a second flexible portion, with the second flexible portion being stiffer than the first flexible portion.

Another aspect of the present disclosure is a catheter apparatus comprising a catheter hub having a catheter tube attached thereto; a needle with a needle tip attached to a needle hub and the needle projecting through the catheter tube with the needle tip projecting distally of a distal opening of the catheter tube; the catheter tube can comprise a catheter body having a wall with an exterior surface, an interior surface, a wall thickness between the exterior surface and the interior surface, and a lumen defined by the interior surface.

The catheter body can comprise: a first portion of the wall thickness formed from a first material with a first stiffness property, the first portion having an inner surface forming at least part of the interior surface of the catheter body and of the lumen and an outer surface forming at least a part of the exterior surface of the catheter body; and a second portion of the wall thickness formed from a second material with a second stiffness property, the second portion having an inner surface and an outer surface.

The second stiffness property of the second material can be greater than the first stiffness property of the first material.

The second portion can be embedded within the wall thickness of the catheter tube or not embedded within the wall thickness of the catheter tube. When the second portion is embedded within the wall thickness of the catheter tube, it is understood that the outer or exterior surface of the second portion is enclosed by the wall thickness of the catheter tube, or enclosed by the first portion. When the second portion is not embedded within the wall thickness of the catheter tube, it is understood that the exterior surface, the interior surface, or both the exterior surface and the interior surface of the second portion is exposed and not covered by the wall thickness of the catheter tube, or by the first portion.

When the second portion is not embedded within the wall thickness of the catheter tube, (i) the inner surface of the second portion forms another part of the interior surface of the catheter body and of the lumen, (ii) the outer surface of the second portion forms another part of the exterior surface of the catheter body, or (iii) the inner surface of the second portion forms another part of the interior surface of the catheter body and of the lumen and outer surface of the second portion forms another part of the exterior surface of the catheter body.

The second portion can have a cross-sectional profile of a width that is generally constant along a length of the catheter tube. The second portion can be a spine or a stiffener when the material of the second portion is stiffer than the material of the first portion.

The second portion can also have a surface that extends lengthwise, or along the length of the catheter tube.

A length of the catheter body can be from about 1.4 cm to about 6.4 cm or from about 8 cm to about 12 cm.

A distal end of the catheter tube can be tapered.

The first material can comprise polyurethane (PUR) and can have a lower stiffness property than the second stiffness property.

The material can comprise fluorinated ethylene propylene (FEP) and can have a lower stiffness property than the second stiffness property.

The first material can comprise polyether block amide (PEBA) and can have a lower stiffness property than the second stiffness property.

The second material can be barium sulfate (BaSO₄).

The second material can alternatively be made from PEEK or PROPELL^{TM}.

In yet other examples, the second material used to make the second portion, or the spine, can be bismuth subcarbonate (Bi₂O₂CO₃) or bismuth oxychloride (BiOCl).

The second material can be fluorinated ethylene propylene (FEP) and the first material has a lower stiffness property. The first material can be PUR.

The first portion and the second portion can extend from or proximate the distal opening of the catheter body towards a proximal end of the catheter body.

The catheter body can have three strips of spaced apart spines each with a stiffness property greater than the first stiffness property used to form the catheter body.

The second portion can be a first spine and the tube body can further comprise a second spine spaced from the first spine.

The second portion can have a cross-sectional profile of a width that increases from a distal end to a proximal end along a length of the catheter tube.

A needle guard having a surface configured to cover the needle tip can be provided with the catheter hub. For example, the needle guard can have a surface located to a side of the needle in the ready to use position and wherein the surface of the needle guard is movable distal of the needle tip in a protective position to cover the needle tip for inadvertent needlesticks.

The needle guard can be located in an interior cavity of the catheter hub in the ready position.

The needle guard can comprise a proximal wall and two arms extending distally of the proximal wall. The two arms can intersect one another in a ready to use position and in a protective position.

The second portion can comprise two or more spaced apart spines.

The two or more spaced apart spines can be embedded within the wall thickness of the catheter tube.

The two or more spaced apart spines can be not embedded within the wall thickness of the catheter tube.

At least one spine can be embedded within the wall thickness of the catheter tube and at least one spine can be not embedded within the wall thickness of the catheter tube.

Catheter tubes described herein can be used with an over-the-needle assembly for catheterization to reduce or minimize kinking by utilizing at least one strip or spine with the tube body that is stiffer than the rest of the tube body and the use of the tube body with the at least one spine can be performed when not involving X-ray or when visual detection of the catheter tube is not needed or required.

Catheter tubes described herein can be used with an over-the-needle assembly for catheterization to reduce or minimize kinking by utilizing at least one strip or spine with the tube body that is stiffer than the rest of the tube body in a tube body length that is longer than a standard catheter tube body. For example, a catheter tube body with the catheter tube as described with at least one spine can have a length of from about 8 cm to about 12 cm, which is longer than standard tube bodies with lengths of from about 1.4 cm to about 6.4 cm. However, a catheter tube body with the catheter tube as described with at least one spine can have a length of standard tube bodies of from about 1.4 cm to about 6.4 cm.

Aspects of the present invention further includes a method of forming a catheter assembly. The method can comprise: forming a catheter hub having a catheter tube attached thereto; forming a needle hub with a needle having a needle tip and projecting the needle through the catheter tube with the needle tip projecting distally of a distal opening of the catheter tube.

The catheter tube can comprise a catheter body having a wall with an exterior surface, an interior surface, a wall thickness between the exterior surface and the interior surface, and a lumen defined by the interior surface

Aspects of the present invention further include a method of using a catheter assembly with a catheter tube having kinking resistant characteristics. The method of using can comprise: placing the catheter tube into a vein, said catheter tube attached to a catheter hub; and wherein the catheter tube comprises a catheter body having a wall with an exterior surface, an interior surface, a wall thickness between the exterior surface and the interior surface, and a lumen defined by the interior surface.

An intravenous catheter device or apparatus in a ready position can have a needle tip of a needle extending out a distal end of a catheter tube for venipuncture. The catheter device or apparatus can be referred to interchangeably as an over-the needle catheter device or a needle device throughout the disclosure.

The catheter device or apparatus can include a needle having a needle tip connected to a needle hub, a catheter hub including a hub body defining an interior cavity, and a catheter tube extending distally of the catheter hub.

The needle hub can couple directly to or in contact with a proximal end of the catheter hub. In other examples, the needle hub may be indirectly coupled to the proximal end of the catheter hub by an intermediate hub (not shown). For example, a third hub as shown in FIGs. 13 and 14 of U.S. Patent No. 8,591,468 may be disposed between the catheter hub and the needle hub and the needle hub spaced from the catheter hub. Contents of the '468 patent are expressly incorporated herein by reference for all purposes.

In the ready position, before placement of the catheter tube into a patient's vein, the needle with the needle tip can project through a lumen or bore of the catheter tube. The needle tip can have a bevel with the bevel facing away from the skin of the patient or upwardly during venipuncture. The upwardly facing bevel of the needle tip can be oriented the same way as the upper portion of the catheter hub body and away from the lower portion that faces the patient's skin.

The needle can project through the lumen of the catheter tube and forms a seal with a distal opening at a distal end of the catheter tube to prevent blood from flowing through the annular space between catheter tube and the exterior of the needle after successful venipuncture.

The distal end of the catheter tube may be tapered inwardly and the opening forming a tight fit around the needle so that when the needle and the catheter tube are inserted together into the patient, the catheter tube does not snag on any tissue, such as the skin and the wall of the vein, during insertion of the needle into the vein.

When the needle punctures the venous wall of the patient and enters the vein, blood may flow into the needle hub through the needle. The blood may flow into an interior cavity of the needle hub and/or a blood collection device or vent plug located at a proximal end of the needle hub. This is known as primary flashback, which is used to indicate proper venous entry.

A needle guard may be positioned inside the interior cavity of the catheter hub. In an example, the needle guard may be a clip type mounted on the needle and slidable on the needle to cover the needle tip. The needle guard may optionally be positioned in a third housing located between the needle hub and the catheter hub, as previously described.

In another example, the needle guard may be a retractable type that retracts the needle and the needle tip into a protective housing, with or without a spring. In still other examples, the needle guard is of the type that moves a barrel or sheath over the needle tip. Where the needle guard is a clip type, a change in profile, such as a crimp or a bulge, may be incorporated proximal of the needle tip for engaging a perimeter defining an opening on the needle guard. In other examples, a tether rather than a change in profile, may be used to prevent the needle guard from displacing distally off of the needle. Exemplary catheter assemblies are shown in U.S. Patent No. 8,333,735, the contents of which are expressly incorporated herein by reference.

In still other examples, a valve and a valve opener can be positioned inside the catheter hub to restrict blood from flowing out the proximal opening of the hub body following removal of the needle and the needle hub from the catheter hub after successful venipuncture. The valve can have one or more slits defining one or more flaps. The valve opener can advance distally into the valve to open the valve by inserting a male Luer tip into the catheter hub to push the valve opener in the distal direction. Aspects of the valve and valve opener are discussed in U.S. Patent No. 8,333,735, previously incorporated by reference. Valves and valve openers are also described in U.S. Pub. No. 2018/0214673, the contents of which are expressly incorporated herein by reference.

A catheter hub can include a tab positioned on an upper portion of the catheter hub. The tab can be used as leverage during insertion and/or removal of the needle and needle hub. The tab can be located at the "upper portion" of the catheter hub, which can be understood as facing away from the skin when used with or on a patient. A registration slot can be located on or with the catheter hub opposite the tab. The registration slot can be configured to receive a rib or projection on the needle hub to facilitate alignment and orientation of the needle and the needle hub with the catheter hub. The registration slot can be located at the external threads of the catheter hub. If the tab is omitted, the upper portion is understood to be the portion that faces up or away from the patient's skin.

The catheter hub can have a hub body and an interior cavity defined by the wall surface of the hub body. The catheter hub can further include a catheter tube in fluid communication with the interior cavity of the hub body.

The catheter tube can attach to a distal section of the hub body using conventional means, such as with a metal bushing. The metal bushing can act as a wedge to secure a proximal end of the catheter tube to the hub body. In other embodiments, the catheter tube can communicate with the interior cavity of the hub body as well as a fluid port extending from a side of the hub body.

A flexible valve, typically in a cylindrical configuration, can be located inside the catheter hub to control fluid flow through the fluid port, if the fluid port is incorporated. The fluid port can extend at an angle from the axis of the hub body or perpendicular to the axis of the hub body. The hub body can have a proximal inlet at a proximal section and with a female Luer taper for receiving a male Luer tip, such as a male infusion line, a syringe, or a male Luer adaptor. The proximal section may also include external threads to securely engage with threads on male Luer lock fittings or tip of a syringe, also known as a Luer lock.

The catheter hub may also include a tab positioned on the hub body (between the proximal section and the distal section of the hub body) to aid in gripping and/or guiding the needle device when inserting the needle device into the patient's vein. Hereinafter, the upper portion of the catheter hub or hub body can be understood as referring to where the tab is located. Further, the upper portion is understood to mean, elevation-wise, the portion of the catheter hub, catheter device, or hub body that is above the pair of wings or above a lower hub portion configured for contacting a patient's skin.

The needle device in a ready to use position should have the bevel of the needle tip facing upwardly, such as being arranged in the similar orientation as the upper portion of the catheter hub if the catheter hub extends directly over the bevel, and away from the skin of the patient. The tab may be used as a reference point to orient the needle device relative to the patient's skin and the puncture site.

With the bevel of the needle being oriented along the same upwardly direction as the upper portion of the catheter hub where the tab is located, the location of the tab can be used as an indicator of the location of the bevel when inserting the needle device into the patient's vein, and when mounting and securing the catheter hub to the patient after successful venipuncture.

The tab can have a rectangular shape with smooth edges. However, the tab may embody any shape and thickness so long as there is sufficient rigidity to provide a leverage point for the user to push against. Grooves or small protrusions may be formed on the surfaces of the tab to aid in gripping or holding the tab. The location of the tab can also be used to indicate the stiffened region of the catheter tube, such as the upper portion of a tube body.

A pair of wings may extend laterally of the hub body to provide additional surface areas for supporting the catheter hub against the patient. In some embodiments, the catheter hub may also be equipped with a septum or a valve (not shown) located inside the interior cavity of the hub body or adjacent the proximal inlet of the hub body to limit or restrict fluid flow across the catheter hub.

A catheter tube can include a catheter body or tube body with an exterior or outer surface and an interior or inner surface defining a lumen or catheter lumen. The catheter lumen can be in fluid communication with the catheter hub, such as with the interior cavity of the catheter hub. The tube body can have a wall thickness between the exterior surface and the interior surface.

The diameter of the catheter lumen can be sufficiently large to surround the needle and for the delivery of fluid at a desired flow rate to and/or from the patient after successful venipuncture. The inside diameter or the catheter lumen proximal of the distal end is slightly larger than a diameter of the needle. The catheter body can have a tapered portion at a distal end or distal tip of the catheter body and the proximal end can be coupled indirectly or directly to the catheter body by, for example, a metal bushing or some other attachment means such as adhesive.

The catheter body can have a wall thickness between an outer surface or outer boundary of the catheter body and the interior surface defining the catheter lumen. The wall thickness may be constant along a length of the catheter body proximal of the tapered portion and decreases at the tapered portion towards the distal end of the catheter body. Said differently, a diameter of the outer surface of the catheter body can be substantially the same along the length of the catheter body proximal of the tapered portion and decreases at the tapered portion towards the distal end of the catheter body.

A distal lumen opening or distal opening is defined at the distal end of the catheter body. In an embodiment, the diameter of the distal lumen opening is smaller than a nominal diameter of the catheter lumen so that the distal opening of the distal end has a form fitting around the needle. The distal end can have a seal around the needle shaft. The distal lumen opening can be slightly smaller than a diameter of the needle to form a seal with the needle. When the needle is removed after successful venipuncture or moves proximally so that at least part of the bevel is within the lumen, the seal between the distal lumen opening and the needle can be terminated to allow blood to flow into the catheter lumen indicating that the catheter tube has successfully penetrated the vein providing access to the patient's vasculature. This is known as secondary flashback.

The catheter body can comprise a first portion formed with a first material and a second portion formed with a second material connected together to form the tubular structure. The tubular structure of the catheter body formed with at least the first portion and the second portion can have a uniform exterior surface and a uniform interior surface. Both the first material and the second material can be flexible. However, between the two, the second material can be harder or stiffer than the first material. For example, the second material can have a stiffness property that is higher in value than the stiffness property of the first material.

Both the first portion made from a first material and the second portion made from a second material can each form an arcuate shaped structure having a concave inner surface and a convex outer surface. However, the sides of the first and second portions can have any shape so that the overall shape of the first portion and of the second portion, aside from having arcuate inner and outer surfaces, can have any shape.

A length of the first portion and a length of the second portion can extend parallel to the axis of the catheter tube. The sides of the first portion can be connected to the sides of the second portion to cooperatively form the catheter tube. That is, both the first portion made of a first material and the second portion made of a second material, which is different from the first material, can extend longitudinally side by side and run parallel to the axis of the catheter tube.

The concave inner surface of the first portion and the concave inner surface of the second portion can jointly form the catheter lumen, and the convex outer surface of the first portion and the convex outer surface of the second portion can jointly form the outer surface or outer boundary of the catheter body.

In other examples, there can be multiple first portions and multiple second portions joined together to form the catheter tube of the present invention.

In some embodiments, only the concave inner surface of the second portion made of a second material and the convex surface of the second portion form the catheter lumen and the outer surface of the catheter body, respectively, while the first portion made from a first material is embedded within the inner and outer surfaces, e.g., within the wall thickness, of the second portion. In other embodiments, only the concave inner surface of the first portion made of a first material and the convex surface of the first portion form the catheter lumen and the outer surface of the catheter body, respectively, while the second portion made from a second material is embedded within the inner and outer surfaces, e.g., within the wall thickness, of the first portion.

The second portion made from a second material can have a stiffness (k) greater than the stiffness of the first portion made from a first material. Accordingly, where a catheter tube has both a first portion and a second portion, the second portion forms a region of the catheter tube that is stiffer than other portion or portions of the catheter tube not formed by the second material. As a result of the stiffened region of the catheter body formed by the second portion, the overall stiffness of the catheter body can increase compared to a catheter tube made entirely from the first material. Accordingly, the modulus of elasticity or Young's modulus (E), which is proportional to stiffness, of the catheter body is also greater than a catheter body without the stiffened region, or where that catheter body is made entirely from the same first material without at least one strip of a relatively stiffer material.

The shape of the second portion may also affect the overall stiffness of the catheter body. For example, the overall stiffness of the catheter body can increase by an increase in the moment of inertia of the second portion. In an example, an increase in moment of inertia may be achieved by increasing the cross-sectional area of the spine or by changing the shape of the spine. When the stiffness of the second portion is increased, the overall modulus of elasticity of the catheter tube can be increased. Again, the stiffness of the second portion can be increased by changing the shape and/or the width of the second portion.

The increase in stiffness of the catheter body can require a larger force to deflect the catheter tube, thereby reducing the likelihood of kinking. Thus, the increased stiffness of the catheter body featuring a second portion made of a second material that is stiffer than a first material to make a first portion of the catheter tube allows use of a relatively longer catheter tube while maintaining a diameter catheter body that is similar or the same as a catheter tube with a catheter body with a first portion only, without a second portion.

In some examples, by incorporating a second portion with a first portion to form a tube body of a catheter tube, the length of the catheter tube can lengthen compared to a standard catheter tube and can range from about 8 cm to about 12 cm. Optionally, the catheter tube of the present disclosure having a first portion 152 and a second portion 155 can also be used for shorter length catheter tubes or for standard length catheter tubes, for example catheter tubes with lengths of from about 1.4 cm to about 6.4 cm.

By utilizing a second portion made of a second material that is stiffer than the first material of a first portion, this can allow the first portion to be made from a softer, more flexible, less stiff material, thereby reducing the probability of causing damage to the inside surface of the wall of the vein from contact.

In some examples, the first portion can form the lower portion of the catheter body while the second portion can form the upper portion, elevation-wise, of the catheter body. This arrangement can be useful for certain catheterization, such as for a shallow venipuncture.

The catheter tube of the present invention having a first portion made of a first material with a first hardness and a second portion made of a second material with a second hardness can be used to limit or prevent tube kinking, can be used to make relatively longer catheter tube lengths compared to standard catheter tubes made from a single material formed throughout, and/or used for accessing a patient's vein but not to facilitate X-ray or image capture of the catheter tube. The second material for forming the second portion can be a single strip of second material or can comprise two or more spaced apart strips. Each strip can comprise a surface and a cross-sectional area. The area can have a regular shape or an irregular shape.

The second portion made from a second material stiffer than the first material of the first portion can be called a spine or a catheter spine. As discussed above, the spine, i.e., the second portion made from the second material with relatively stiffer property, can help to prevent or resist kinking of the catheter tube, which if occurs can block fluid flow through the catheter lumen. For example, after successful venipuncture, a kinked catheter tube can block or slow the flow of IV solution to the patient. Thus, it is preferable to use a catheter tube that is resistant to kinking. The catheter tube of the present disclosure, with a first portion having a first material and a second portion having a second material, which is stiffer than the first material, is resistant to kinking.

In an example, the spine, or the second material of the second portion, has a constant cross-sectional profile and extends longitudinally along an upper portion of the catheter body, on the same side as the tab of the catheter hub. That is, an exemplary embodiment has a spine formed as a narrow strip with a substantially constant cross-sectional profile extending between the proximal end of the catheter tube and the distal end of the catheter tube.

In some embodiments, the spine forms a narrow strip that does not have a constant cross-sectional profile along the length of the catheter body.

In other embodiments, the spine has a variable cross-sectional profile along a length of the catheter body. For example, the distal portion of the catheter tube can be provided with a relatively narrow cross-sectional profile and the cross-sectional profile can increase in width as the length extends in the proximal direction. Still further, rather than having tapered sidewalls for the spine, the sidewalls can vary between straight, taper, undulating, tapering outwardly, etc.

The first portion is joined together at the sides of the catheter spine to jointly form a seamless and smooth outer surface of the catheter body. This allows the catheter tube to avoid snagging or shearing tissues when the catheter tube is inserted into the patient to access the vein and when feeding the catheter tube to the desired location inside the vein. The first portion and the catheter spine may also be joined together to form a seamless catheter lumen or inner surface. For example, the spine can be co-extruded with the first portion to form a seamless inner surface and outer surface tube body of a catheter tube.

In an embodiment, the spine is oriented upwardly similar to the top side or upper portion of the catheter body. Said differently, the spine can make up the upper portion of the catheter body or catheter tube and the first portion can make up the remainder or at least the lower portion of the catheter body, elevation-wise.

The cross-sectional profile of the spine of the catheter body can occupy about 25 to about 180 degrees of the arc of the catheter body and the first portion can occupy the remaining portion of the catheter body. The spine may occupy more or less of the catheter body depending on the outer diameter of the catheter body and the desired overall stiffness of the catheter body. That is, the width of the cross-sectional profile and the shape of the spine can determine the stiffness and, consequently, the desired length of the catheter body.

To increase the stiffness of a catheter tube, a material of the spine can be chosen, for example, to be stiffer than a typical material used for the catheter tube, such as stiffer than the remaining material used to form the catheter tube. The relatively stiffer material selected for the spine can be used to form the entire catheter tube but more preferably only portions of the catheter tube while the remaining portions can be formed using typical or conventional catheter tube materials.

In an example, the body of the catheter tube has at least two different materials used to form the length of the tube body, such as 50% or more of the length of the tube body. In an example, the material of the spine should be harder than fluorinated ethylene propylene copolymer (FEP) material, which is typically used for a standard single material catheter body. Another exemplary material that is usable to form the first portion is polyurethane (PUR). In some examples, the second portion for forming a spine can be made from FEP while the first portion for forming the remaining portion of the tube body is made from PUR.

In a particular example, barium sulfate (BaSO₄) can be used to form a spine of a tube body. Thus, as a particular example, a catheter tube having a tube body with a lumen can be formed using FEP or PUR material with a BaSO₄ material, and wherein the BaSO₄ material is used to form a spine, or the second portion, that runs lengthwise of the tube body and the FEP or PUR material forming the balance of the tube body of a catheter tube, which can be referred to as the first portion.

The first portion can be made from a softer common catheter material, which can include polyurethane (PUR) or FEP. In an example, the BaSO4 material is mixed with an effective amount of a polyether block amide (PEBA) or other compatible polymer materials to facilitate bonding with the first material, such as to facilitate bonding with the FEP or PUR material. Any suitable biocompatible material can be used for the second portion as long as the material of the second portion used to form the spine has a greater stiffness than the material used to form the first portion.

The catheter tube can be manufactured by a co-extrusion manufacturing process. The second material used to form the spine can be embedded within the inner and outer surfaces of the tube body or can be co-extruded to form at least part of the exterior surface, the interior surface, or both the interior and exterior surfaces of the tube body.

In still other examples, the tube body can have multiple spines or multiple spaced apart second portions that are formed with a material making up the first portion to form the tube body of a catheter tube in accordance with aspects of the present catheter tubes.

The multiple spines can be embedded or not embedded within the inner and outer surfaces of the tube body or there can be spines that are embedded and spines that are not embedded within the inner and outer surfaces of a tube body.

The increased stiffness of the tube body of a catheter tube when one or more strips of spines are incorporated allows for a longer catheter tube to be utilized. The catheter body can be made stiffer, at least along the section or space occupied by the spine, to decrease the likelihood of bending or kinking. Because the lower portion of the catheter body having first and second portions can be made from a softer material, the softer material to form the first portion and the relatively stiffer or harder material to form the spine of the second portion, the likelihood of injuries caused by contact made between the lower portion of the catheter body and the interior wall tissues of a vein can be minimized.

For a catheter tube utilized with a catheter hub in which a spine is formed along an upper portion of the tube body and a first portion is formed with a more flexible material or less stiff material along a lower portion of the tube body, the catheter tube can be advanced into the vein after successful venipuncture with the needle removed from the catheter tube. During advancement of the catheter tube, the distal tip of the tube body may encounter the inside wall of the vein. A reaction force by the inside wall counters the driving action of the catheter tube is applied to the catheter tube by the venous wall. The reaction force applied to the catheter tube can cause the catheter tube to deflect and the angle of deflection to increase.

The reaction force could cause the lower portion of the catheter body to bend upwardly, resulting in the lower portion being under tension and the upper portion of the catheter body, such as the second portion or the spine, experiencing at least some compression. However, because the catheter tube is stiffer when one or more spines are incorporated with the tube body in accordance with aspects of the present invention, the upward deflection is limited by the rigidity of the spine thereby allowing the distal tip of the catheter tube to advance further into the vein without bending too far or too much upwardly, such as being bent completely upwardly, to contact the opposite side if the venous wall and possibly kink.

For a typical catheter tube, if the deflection is too large when encountering the inside wall of a vein, the catheter tube can form a tight bend or a kink, and as a result, reduce or prevent the flow of fluid through the catheter lumen. If the stiffness or Young's modulus of the catheter tube is increased, such as by incorporating a spine of the present disclosure, a larger force would be required to bend the catheter tube and therefore reduce the likelihood of a kink formed in the catheter tube. The stiffness of the catheter tube can be adjusted by changing the width or the shape of the cross-sectional profile of the spine, or decrease the number of spines used with the tube body, or both.

In still other examples, the relative stiffness between the second portion and the first portion can be selected based on selection of materials. The materials can be selected so that the material of the second portion compared to the material of the first portion can have a stiffness value as a ratio of from about 1.05 to 1.8 of second material stiffness to first material stiffness. In still other examples, the stiffness value as a ratio is selected to be greater than about 1.8 of second material stiffness to first material stiffness. For example, the second material stiffness can have a Young's modulus (E) value of 3.7 MPa and the first material stiffness can have a Young's modulus (E) value of 2.46 MPa and the ratio of second material stiffness to first material stiffness is 1.5.

In one embodiment, the width of the cross-sectional profile of the spine can be generally constant lengthwise from proximate the distal tip of the catheter body and extending towards the proximal end of the catheter body. The spine may or may not extend to the very proximal end of the catheter body. In another embodiment, the spine may have a width or a cross-sectional profile that increases or varies from a point at the distal end of the catheter body or tube, such as a point just proximal of the tapered portion at the distal end or starting from the distal opening, and extending towards the proximal end of the catheter body or tube. In the embodiment with the increasing cross-sectional width, the stiffness of the tube body increases from the distal end of the tube body to the proximal end of the tube body. In still other examples, the distal most point of the spine can originate proximal of the tapered portion, and up to several millimeters proximal of the tapered portion.

In addition to the location of the spine, the shape of the spine can also contribute to the stiffness of the catheter tube. In one example, the spine has an arcuate shaped structure. The arcuate shaped structure of the spine and the cylindrical shape of the catheter tube as a whole allows the catheter tube to extend in a straight configuration along a lengthwise direction. The stiffness of the spine and of the catheter tube can increase by increasing the width of the cross-sectional profile of the arcuate shaped spine, which has a larger width along the outer surface of the spine than the inside surface of the spine. As the width of the cross-sectional profile of the spine increases, so does the height of the arcuate shaped spine, thereby dramatically increasing the moment of inertia of the spine.

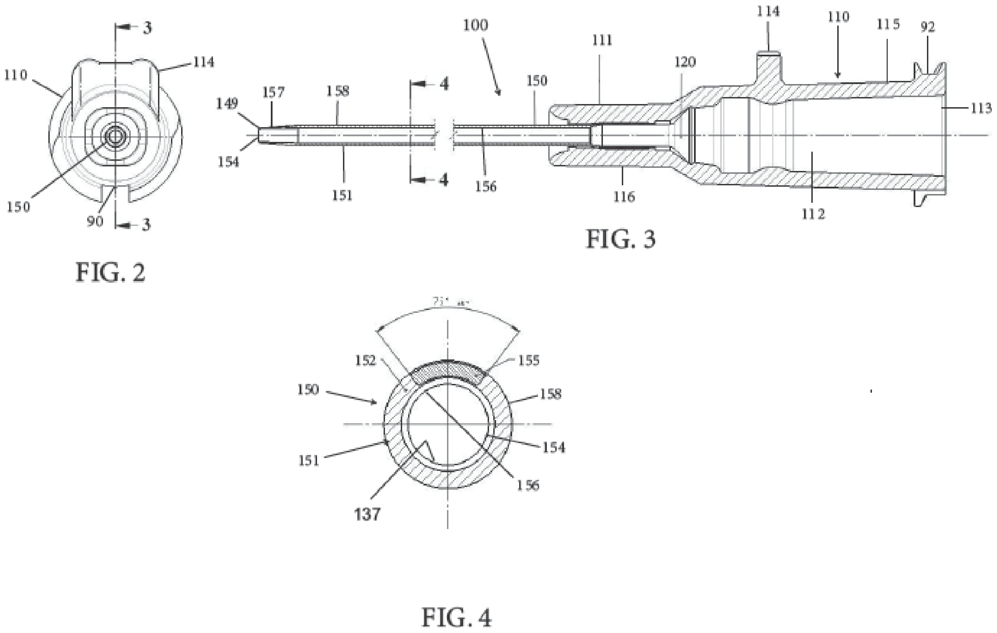

As a comparison, when taking a ¼ section of the catheter tube and placing it on a flat surface compared to taking a ½ section of the catheter tube and placing it on a flat surface, the height of the ½ section is higher. Thus, by increasing the width of the cross-sectional profile of the spine, the height can also increase. The increase in moment of inertia also increases the stiffness of the spine. Simply stated, the stiffness of the catheter tube can be adjusted by the shape of the spine. For example, the catheter tube can have a first stiffness when a spine having a cross-sectional profile of a first width and the catheter tube can have a second stiffness by changing the shape of the cross-sectional profile to a second width, which is larger than the first width. In yet another example, the stiffness can increase by changing the angles of the two sidewalls of the arcuate shaped spine. For example, looking at the sidewalls of the spine of FIG. 4, the sidewalls can taper outward when extending from the exterior surface to the interior surface so that the interior arcuate surface is wider than the exterior arcuate surface.

A catheter tube can include one or more spines that are embedded inside the wall thickness of the first portion. One, two, three or more than three spines contemplated. When more than one embedded spine are incorporated, such as two spines or more, the spines can be equally spaced or unequally spaced from one another. The spines can be spaced away from the catheter lumen and the outer surface of the catheter body. The embedded spines can be enclosed or encased between the inner and outer surfaces of the tube body.

A catheter tube can have both types of spines, such as one or more embedded spines and one or more spines that are not embedded.

The shape of a spine can be oval, circular, rectangular, or any other regular or irregular shape. A spine can run lengthwise and extend between the proximal end and the distal end of a catheter body, including to the proximal-most and distal-most ends of the proximal and distal ends. The material of a spine that is embedded can be the same material or a material that is softer than the material used to form the spine that is not embedded. The spine that is not embedded has a surface that is exposed along the exterior surface of the tube body, along the interior surface of the tube body, or both.

The material of the first portion can be softer than the material of both the spines that are embedded within the tube body, which are spaced from one another and have surfaces that are entirely within the interior and exterior surfaces of the tube body, and the spine that is not embedded within the tube body, which has at least one surface that is exposed along the exterior surface of the tube body, along the interior surface of the tube body, or both.

In an example, the spine that is not embedded is made from BaSO₄, and the first portion is made from polyurethane. Alternatively, the first portion is made from silicone. In yet other examples, the first portion is made from polyethylene. In still other examples, the first portion is made from a compound, such as from Teflon/PTFE.

The spines that are embedded within the tube body can be made from BaSO₄. If BaSO4 is used, the material for the spine can be blended with an affective amount of PEBA to facilitate bonding with the material of the first portion. BaSO₄ has properties that are sufficient for increasing the stiffness of the catheter tube and can provide X-ray visibility as well. Aspects of the present disclosure is directed to the use of the disclosed catheter tubes to limit or prevent kinking and to enable the fabrication of extended length tube bodies compared to tube bodies made from a single material or from a uniform blended composite.

In some examples, the relatively stiffer material used to form or make the spine is PEEK or PROPELL^{TM}. In yet other examples, the second material used to make the second portion, or the spine, is bismuth subcarbonate (Bi₂O₂CO₃) or bismuth oxychloride (BiOCl).

When multiple strips of a second portion are incorporated with a first portion to form a tube body of a catheter tube, the multiple strips of the second portion can be made from the same material or from different materials. For example, in a catheter tube embodiment with two embedded spines and one non-embedded spine, BaSO₄ can be used to make the non-embedded spine and Bi2O2CO3 can be used to make the embedded spines.

The spines can increase the overall stiffness of the catheter tube. The spines can be of the embedded type or the non-embedded type or both. In some examples, the tube body can have multiple spine types, such as two or more embedded spines and two or more non-embedded spines. When incorporated, the one or more spines should be positioned so that stiffer region of the catheter tube is along the upper portion of the tube body. The spines may also be located away from the distal tip and the tapered portion of the catheter body to ensure the distal tip of the catheter body remains a softer first portion. The catheter tube can be made by a co-extrusion process.

In an example, the three embedded spines can be made from a BaSO₄ material and can be used for X-ray visibility and optical transparency. The non-embedded spine can be relatively larger, such as having a greater girth or width, than the embedded spines to increase the stiffness of the tube body along the upper portion of the tube body. This arrangement has all the advantages of similar catheter bodies with a relatively stiffer upper portion described elsewhere herein.

A catheter tube can include two non-embedded spines spaced from each other by a first portion. A non-embedded spine, or a strip of material having a different stiffness property than the material used to form the tube body, is understood as one that has an exposed interior surface, exterior surface, or both exposed interior and exterior surfaces.

Two non-embedded spines can be located away from the upper portion of a catheter body, such as being located but along a horizontal median line passing through the tube body. In such an embodiment, the softer first material is incorporated at both an upper portion of the catheter body and at a lower portion of the catheter body. Therefore, the lower portion of the catheter body that can contact the interior surface of the venous wall during advancement of the catheter tube would be the softer first portion made of the first material to minimize potential injury or damage to the venous wall.

In an embodiment with two non-embedded spines that are located away from the upper portion of a catheter body, the interior concave surfaces of the two spines and the interior concave surfaces of the first portion and the outer convex surfaces of the two spines and the outer convex surfaces of the first portion can jointly form the exterior of the catheter tube. In one embodiment, the spines of the second portion can be made from a BaSO₄ material and the first portion can be made from a polyurethane material. Optionally, an effective amount of PEBA can be incorporated with the BaSO₄ material to facilitate bonding.

The advantages of a catheter tube with at least one spine made from a material having increased stiffness from the remaining portion of the tube body and located away from the lower portion of the catheter tube include a stronger resistance to bending and kinking while maintaining a soft lower portion that may contact the interior surface of the venous wall during advancement of the catheter tube after venipuncture. The hardness and thus the stiffness of the spine can be configured according to the need and application of the catheter device or apparatus or can be used for a standard length over-the-needle catheter, not just for extended length catheter tubes.

Bigger benefits can be derived when using the teachings of the present invention in connection with a relatively longer length catheter tube. Moreover, the increased stiffness of the catheter tube ensures patency, such as an unblocked lumen, thereby preventing infection or phlebitis, and reduction in pain. Another benefit of the increased stiffness is the capability of the catheter tube of the present invention to be advanced deep into a vein without a guidewire, although a guidewire can be used. The catheter tube of the present invention is a kink resistant tube having first and second portions made from two different materials with two different stiffness properties with a softer flexible material of the two specially located to minimize or prevent injury to the venous wall. For example, the softer flexible material can be located along a lower or bottom portion of the catheter body, elevation-wise.

A catheter tube in accordance with aspects of the present invention can comprise a first section or portion made of a first material and a second section or portion made of a second material. The tube body can have an exterior surface and an interior surface defining a lumen. In the present embodiment, the second portion can be a strip embedded into the first portion, such as embedded into the wall thickness of the first portion, between the interior and exterior surfaces. The second portion, which can be called a spine, can have surfaces that are entirely encased within the first portion. The first portion may be made from PUR or PEBA and the second portion may be made from FEP. In still other examples, the first portion may be made from FEP, PUR, or PEBA and the second portion may be made from BaSO₄. The second portion can be located along the upper portion of the tube body, elevation wise.

A catheter tube in accordance with further aspects of the present invention can comprise a first section or portion made of a first material and a second section or portion made of a second material. The tube body has an exterior surface and an interior surface defining a lumen. In the present embodiment, the second portion can comprise three spaced apart strips embedded into the first portion, such as embedded into the wall thickness of the first portion, between the interior and exterior surfaces. The three strips of the second portion, which can be called a spine, can each have surfaces that are entirely encased within the first portion. The first portion may be made from PUR or PEBA and the second portion, such as the three spines, may be made from FEP. In still other examples, the first portion may be made from FEP, PUR, or PEBA and the second portion, such as the three spines, may be made from BaSO₄. The second portion, i.e., the three spines, can be located along the upper portion of the tube body, above a median line passing through the center of the tube body.

Methods of making and of using over-the-needle catheter devices in which the catheter tubes have at least two different portions made from at least two different materials are within the scope of the invention.

### BRIEF DESCRIPTION OF THE DRAWINGS

These and other features and advantages of the present devices, systems, and methods will become appreciated as the same becomes better understood with reference to the specification, claims and appended drawings wherein:
FIG. 1 is a cross-sectional view of an over-the needle catheter device or apparatus in accordance with an embodiment of the present disclosure.
FIG. 2 is a front schematic view of a catheter assembly or hub in accordance with an embodiment of the present disclosure.
FIG. 3 is a cross-sectional schematic view of the catheter hub of FIG. 1 taken at line 3-3, the catheter hub including a catheter tube in accordance with an embodiment of the present disclosure.
FIG. 4 is a cross-sectional schematic view of the catheter tube of FIG. 3 taken at line 4-4, the catheter tube including a first body and a second body.
FIG. 5 is a perspective schematic view of a portion of the catheter tube of FIG. 1.
FIG. 6 is a profile schematic view of the portion of the catheter tube in FIG. 5.
FIGs. 7 and 8 are schematic views of a catheter tube in a vein after venipuncture.
FIG. 9 is a cross-sectional schematic view of a catheter tube in accordance with another embodiment of the present disclosure.
FIG. 10 is a cross-sectional schematic view of a catheter tube in accordance with yet another embodiment of the present disclosure.
FIG. 11 is a cross-sectional schematic end view of a catheter tube in accordance with another embodiment of the present disclosure.
FIG. 12 is a cross-sectional schematic end view of yet another catheter tube in accordance with another embodiment of the present disclosure.

### DETAILED DESCRIPTION

The detailed description set forth below in connection with the appended drawings is intended as a description of the embodiments of an intravenous catheter device, apparatus, and assembly having a catheter tube with a stiffened region provided in accordance with aspects of the present devices, systems, and methods and is not intended to represent the only forms in which the present devices, systems, and methods may be constructed or utilized. The description sets forth the features and the steps for constructing and using the embodiments of the present devices, systems, and methods in connection with the illustrated embodiments. It is to be understood, however, that the same or equivalent functions and structures may be accomplished by different embodiments that are also intended to be encompassed within the spirit and scope of the present disclosure. As denoted elsewhere herein, like element numbers are intended to indicate like or similar elements or features.

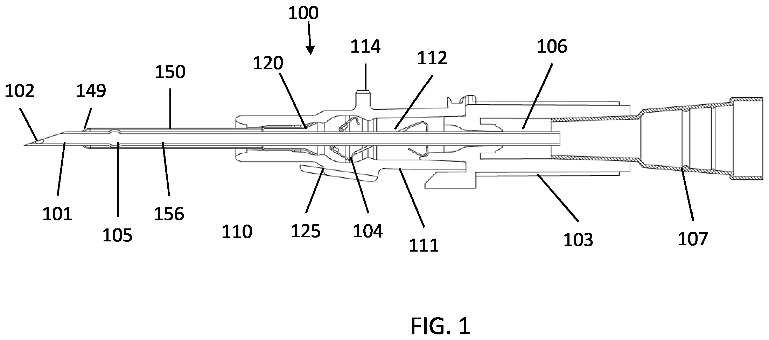

FIG. 1 depicts a cross-sectional view of an intravenous catheter device or apparatus 100, shown in a ready position with the needle tip 102 extending out a distal end for venipuncture. The catheter device or apparatus 100 may also be referred to interchangeably as an over-the needle catheter device, catheter assembly, or a needle device throughout the disclosure. The catheter device, assembly, or apparatus 100 includes a needle 101 having a needle tip 102 connected to a needle hub 103, a catheter hub 110 including a hub body 111 defining an interior cavity 112, and a catheter tube 150 extending distally of the catheter hub 110. The catheter tube may attach to the catheter hub using a ferrule or metal bushing, which is conventional. The needle hub 103 is shown coupled directly to or in contact with a proximal end of the catheter hub 110. In other examples, the needle hub 103 may be indirectly coupled to the proximal end of the catheter hub 110 by an intermediate hub (not shown). For example, a third hub as shown in FIGs. 13 and 14 of U.S. Patent No. 8,591,468 ('468 patent) may be disposed between the catheter hub and the needle hub and the needle hub spaced from the catheter hub. Contents of the '468 patent are expressly incorporated herein by reference for all purposes.

In the ready position, before placement of the catheter tube 150 into a patient's vein, the needle 101 with the needle tip 102 projects through a lumen or bore 156 of the catheter tube 150. The needle tip 102 is shown beveled with the bevel facing away from the skin of the patient or upwardly. The upwardly facing bevel of the needle tip 102 is oriented the same way as the upper portion of the catheter hub body and away from the lower portion that faces the patient's skin.

The needle 101 projects through the lumen 156 of the catheter tube 150 and forms a seal with a distal opening 149 at a distal end of the catheter tube 150 to prevent blood from flowing through the annular space between catheter tube 150 and the exterior of the needle 101 after successful venipuncture. The distal opening 149 at the distal end of the catheter tube 150 may be tapered inwardly and the opening forming a tight fit around the needle so that when the needle 101 and the catheter tube 150 are inserted together into the patient, the catheter tube 150 does not snag on any tissue, such as the skin and the wall of the vein, during insertion of the needle 101 into the vein. When the needle 101 punctures the venous wall of the patient and enters the vein, blood may flow into the needle hub 103 through the needle 101. Blood may flow into an interior cavity 106 of the needle hub 103 and/or a blood collection device or vent plug 107 located at a proximal end of the needle hub 103. This is known as primary flashback, which is used to indicate proper venous entry.

A needle guard 104 may be positioned inside the interior cavity 112 of the catheter hub 110. In an example, the needle guard 104 may be a clip type mounted on the needle 101 and slidable on the needle 101 to cover the needle tip 102. For example, the needle guard 104 can have a surface located to a side of the needle in the ready to use position of FIG. 1 and wherein the surface is movable distal of the needle tip in a protective position to cover the needle tip for inadvertent needlesticks. The needle guard 104 may optionally be located substantially externally of the catheter hub, such as be positioned in a third housing located between the needle hub and the catheter hub, as previously described with reference to the '468 patent. In another example, the needle guard may be a retractable type that retracts the needle 101 and the needle tip 102 into a protective housing, with or without a spring. Where the needle guard is a clip type, a change in profile 105, such as a crimp or a bulge, may be incorporated proximal of the needle tip 102 for engaging a perimeter defining an opening on the needle guard 104. In other examples, a tether rather than a change in profile may be used to prevent the needle guard from displacing distally off of the needle. Exemplary catheter assemblies are shown in U.S. Patent No. 8,333,735, the contents of which are expressly incorporated herein by reference.

In still other examples, a valve and a valve opener can be positioned inside the catheter hub 110 to restrict blood from flowing out the proximal opening of the hub body following removal of the needle and the needle hub from the catheter hub after successful venipuncture. The valve can have one or more slits defining one or more flaps. The valve opener can advance distally into the valve to open the valve by inserting a male Luer tip into the catheter hub to push the valve opener in the distal direction. Aspects of the valve and valve opener are discussed in U.S. Patent No. 8,333,735, previously incorporated by reference.

FIG. 2 illustrates a front view of the catheter hub 110 of FIG. 1, shown without the needle 101 and the needle hub 103, as seen from a distal end or the catheter tube end towards a proximal end of the catheter hub. The catheter hub 110 includes a tab 114 positioned on an upper portion of the catheter hub 110. The tab 114 can be used as leverage during insertion and/or removal of the needle and needle hub. The tab 114 is shown located at the "upper portion" of the catheter hub 110, elevation-wise, which will be described in further detail below with reference to FIG. 3. A registration slot 90 is located on the catheter hub opposite the tab 114. The registration slot 90 is configured to receive a rib or projection on the needle hub to facilitate alignment and orientation of the needle and the needle hub with the catheter hub. The registration slot 90 can be located at the external threads of the catheter hub.

FIG. 3 is a cross-sectional side view of the catheter hub 110 taken along line 3-3 of FIG. 2. The catheter hub 110 has a hub body 111 with a wall and an interior cavity 112 defined by the interior wall surface of the hub body. The catheter hub 110 further includes a catheter tube 150 in fluid communication with the interior cavity 112 of the hub body 111. As shown, the catheter tube 150 is attached to a distal section 116 of the hub body 111 using conventional means, such as with a metal bushing 120. The metal bushing 120 can act as a wedge to secure a proximal end of the catheter tube 150 to the hub body 111. In other embodiments, the catheter tube 150 communicates with the interior cavity 112 of the hub body 111 as well as a fluid port extending from a side of the hub body 111. A flexible valve, typically in a cylindrical configuration, can be located inside the catheter hub to control fluid flow through the fluid port, if the fluid port is incorporated. The fluid port can extend at an angle from the axis of the hub body 111 or perpendicular to the axis of the hub body 111. The hub body 111 has a proximal inlet or proximal opening 113 at a proximal section 115. The proximal section at the opening 113 can have or can incorporate a female Luer taper for receiving a male Luer tip, such as a male infusion line, a syringe, or a male Luer adaptor. The proximal section 115 may also include external threads 92 to securely engage with threads on male Luer lock fittings or tip of a syringe, also known as a Luer lock.

The catheter hub 110 may also include a tab 114 positioned on the hub body 111 (between the proximal section 115 and the distal section 116 of the hub body 111) to aid in gripping and/or guiding the needle device when inserting the needle device into the patient's vein. Hereinafter, the upper portion of the catheter hub or hub body 111 is where the tab 114 is located. If a tab 114 is omitted, the upper portion is understood to be the portion that faces up or away from the patient's skin. Further, the upper portion or upward direction is understood to mean, elevation-wise, the portion or direction of the catheter hub, catheter device, or hub body that is above the pair of wings 125 (FIG. 1) or above a lower hub portion configured for contacting a patient's skin.

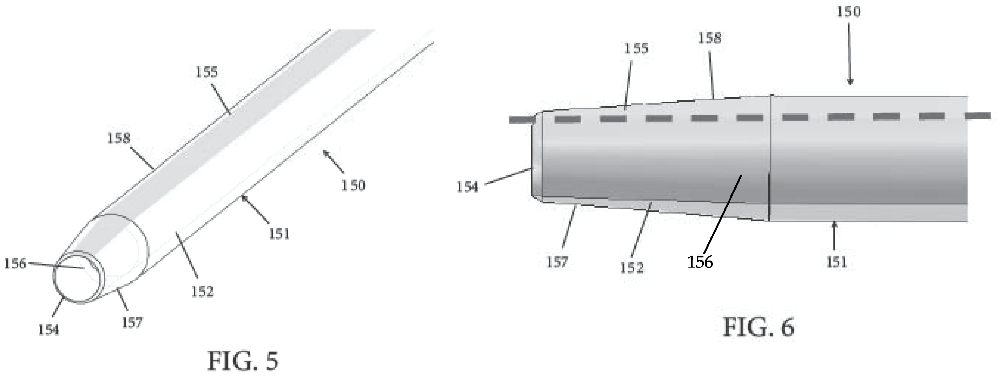

As shown in FIG. 1, in the needle device ready to use position, the bevel of the needle tip 102 faces upwardly, such as towards the upper portion of the catheter hub 110 if the catheter hub extends directly over the bevel, and away from the skin of the patient. The tab 114 may be used as a reference point to orient the needle device relative to the patient's skin and the puncture site. With the bevel of the needle being oriented along the same upward direction as the upper portion of the catheter hub where the tab 114 is located, the location of the tab can be used as an indicator of the location of the bevel when inserting the needle device into the patient's vein, and when mounting and securing the catheter hub 110 to the patient after successful venipuncture. As shown, the tab 114 has a rectangular shape with smooth edges. There can be undulating surfaces incorporated on one or more of the edges. However, the tab 114 may embody any shape and thickness. Grooves or small protrusions may be formed on the surfaces of the tab to aid in gripping or holding the tab 114. As further discussed with reference to FIGs. 4-6 below, the location of the tab 114 can also be used to indicate the stiffened or reinforced region of the catheter tube 150.

A pair of wings 125 (see FIG. 1) may extend laterally of the hub body 111 to provide additional surface areas for supporting the catheter hub 110 against the patient. In some embodiments, the catheter hub 110 may also be equipped with a septum or a valve (not shown) located inside the interior cavity 112 of the hub body 111 or adjacent the proximal inlet 113 of the hub body 111 to limit or restrict fluid flow across the catheter hub 110.

Referring now to FIGs. 4-6 and with continued reference to FIGs. 1 and 2, the catheter tube 150 includes a catheter body or tube body 151 with an exterior or outer surface 158 and an interior or inner surface 137 defining a lumen or catheter lumen 156 in fluid communication with the catheter hub. The catheter tube 150 of the present embodiment, as well as other catheter tubes of the present application, is/are usable with the catheter hubs described elsewhere herein.

The tube body 151 has a wall thickness between the exterior surface 158 and the interior surface. The diameter of the catheter lumen 156 is sufficiently large to surround the needle 101 and for the delivery of fluid at a desired flow rate to and/or from the patient after successful venipuncture. As shown, the inside diameter or the catheter lumen 156 proximal of the distal end or distal opening 149 is slightly larger than a diameter of the needle 101. The catheter body 151 has a tapered portion 157 at a distal end or distal tip of the catheter body 151 and the proximal end can be coupled indirectly or directly to the hub body 111 by, for example, a metal bushing 120 or some other attachment means such as adhesive.

The catheter body or tube body 151 has a wall thickness between an outer surface or outer boundary 158 of the catheter body 151 and the interior surface defining the catheter lumen 156. The wall thickness may be constant along a length of the catheter body 151 proximal of the tapered portion 157 and decreases at the tapered portion 157 towards the distal opening 149 at the distal end of the catheter body 151. Said differently, a diameter of the outer surface 158 of the catheter body 151 is substantially the same along the length of the catheter body 151 proximal of the tapered portion 157 and decreases at the tapered portion 157 towards the distal opening 149 at the distal end of the catheter body 151.

A distal lumen opening or distal opening 154 is defined at the distal end of the catheter body 151. In an embodiment, the diameter of the distal lumen opening 154 is smaller than a nominal diameter of the catheter lumen 156 so that the distal opening 154 of the distal end 149 has a form fitting around the needle. As shown, the distal lumen opening 154 is slightly smaller than a diameter of the needle to form a seal with the needle. When the needle is removed after successful venipuncture or moves proximally so that at least part of the bevel is within the lumen 156 of the tube body 151, the seal between the distal lumen opening 154 and the needle is terminated to allow blood to flow into the catheter lumen 156 indicating that the catheter tube 150 has successfully penetrated the vein providing access to the patient's vasculature. This is known as secondary flashback.

The catheter body 151 comprises a first portion 152 formed with a first material and a second portion formed with a second material 155 connected together to form the tubular structure. The tubular structure of the catheter body formed with at least the first portion 152 and the second portion 155 can have a uniform exterior surface and a uniform interior surface. Both the first material and the second material can be flexible. However, between the two, the second material can be harder or stiffer than the first material. For example, the second material can have a stiffness property that is higher in value than the stiffness property of the first material. Thus, a catheter body 151 in accordance with aspects of the present invention can have a stiffness along an upper portion or upward direction of the catheter body that is stiffer or has a higher stiffness property than remaining portions of the catheter body 151.

As shown, both the first portion 152 made from a first material and the second portion 155 made from a second material each form an arcuate shaped structure having a concave inner surface and a convex outer surface. However, the sides of the first and second portions can have any shape so that the overall shape of the first portion and of the second portion, aside from having arcuate inner and outer surfaces, can have any shape. A length of the first portion 152 and a length of the second portion 155 extend parallel to the axis of the catheter tube 150. The sides of the first portion 152 are connected to the sides of the second portion 155 to cooperatively form the catheter tube 150. That is, both the first portion 152 made of a first material and the second portion 155 made of a second material, which is different from the first material, extend longitudinally side by side and run parallel to the axis of the catheter tube 150. The concave inner surface of the first portion 152 and the concave inner surface of the second portion 155 join together to form the catheter lumen 156, and the convex outer surface of the first portion 152 and the convex outer surface of the second portion 155 jointly form the outer surface or outer boundary 158 of the catheter body 151. In other examples, there can be multiple first portions and multiple second portions joined together to form the catheter tube of the present invention.

In some embodiments, only the concave inner surface of the second portion 155 made of a second material and the convex surface of the second portion 155 form the catheter lumen 156 and the outer surface of the catheter body 151, respectively, while the first portion 152 made from a first material is embedded within the inner and outer surfaces, e.g., within the wall thickness, of the second portion 155. In other embodiments, only the concave inner surface of the first portion 152 made of a first material and the convex surface of the first portion 152 form the catheter lumen 156 and the outer surface of the catheter body 151, respectively, while the second portion 155 made from a second material is embedded within the inner and outer surfaces, e.g., within the wall thickness, of the first portion 152.

The second portion 155 made from a second material has a stiffness (k) greater than the stiffness of the first portion 152 made from a first material and wherein the second portion is located along the upper portion or upward direction of the tube body 151. Accordingly, where a catheter tube 150 has both a first portion 152 and a second portion 155, the second portion 155 forms a region of the catheter tube that is stiffer than other portions of the catheter tube not formed by the second material. As a result of the stiffened region of the catheter body 151 formed by the second portion 155, the overall stiffness of the catheter body 151 can increase compared to a catheter tube made entirely from the first material. Accordingly, the modulus of elasticity or Young's modulus (E), which is proportional to stiffness, of the catheter body 151 is also greater than a catheter body without the stiffened region.

The shape of the second portion 155 may also affect the overall stiffness of the catheter body 151. For example, the overall stiffness of the catheter body can increase by an increase in moment of inertia of the second portion 155. In an example, an increase in moment of inertia may be achieved by increasing the cross-sectional area of the spine or by changing the shape of the spine. When the stiffness of the second portion 155 is increased, the overall modulus of elasticity of the catheter tube 150 can be increased. Again, the stiffness of the second portion 155 can be increased by changing the shape and/or the width of the second portion.

The increase in stiffness of the catheter body 151 can require a larger force to deflect the catheter tube 150, thereby reducing the likelihood of kinking. Thus, the increased stiffness of the catheter body 151 featuring a second portion 155 made of a second material that is stiffer than a first material to make a first portion 152 of the catheter tube 150 allows use of a relatively longer catheter tube 150 while maintaining a diameter catheter body that is similar or the same as a catheter tube with a catheter body with a first portion only, without a second portion. In an example, the second portion can be located along the upper portion or upward direction of the tube body 151.

In some examples, by incorporating a second portion 155 with a first portion 152 to form a tube body of a catheter tube, the length of the catheter tube can lengthen compared to a stranded catheter tube and can range from about 8 cm to about 12 cm. Optionally, the catheter tube of the present disclosure having a first portion 152 and a second portion 155 can also be used for shorter length catheter tubes or for standard length catheter tubes, for example catheter tubes with lengths of from about 1.4 cm to 6.4 cm.

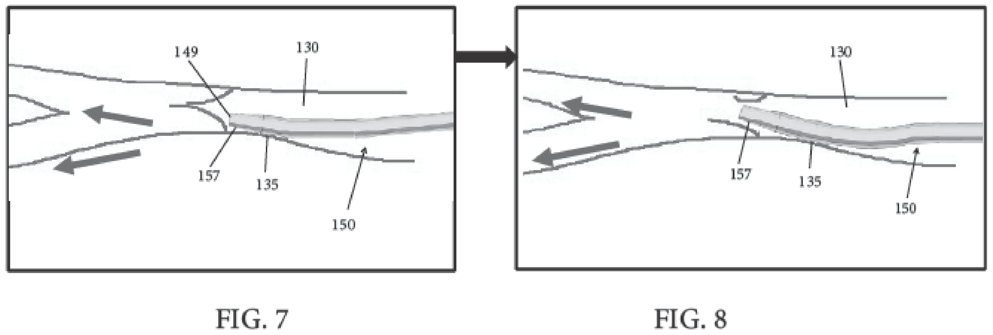

By utilizing a second portion 155 made of a second material that is stiffer than the first material of a first portion 152 to form a catheter tube body having at least two arcuate sections that are joined along two sets of lengthwise edges, this can allow the first portion 152 to be made from a softer, more flexible, less stiff material, thereby reducing the probability of causing damage to the inside surface of the wall of the vein from contact, as discussed further below with reference to FIGs. 7 and 8. In some examples, the first portion 152 can form the lower portion of the catheter body 151 while the second portion 155 can form the upper portion, elevation-wise, of the catheter body 151. The catheter tube of the present invention having a first portion made of a first material with a first hardness and a second portion made of a second material with a second hardness can be used to limit or prevent tube kinking, can be used to make relatively longer catheter tube lengths compared to standard catheter tubes made from a single material formed throughout, and/or used for accessing a patient's vein but not to facilitate X-ray or image capture of the catheter tube. The second material for forming the second portion can be a single strip of second material or can comprise two or more spaced apart strips. Each strip can comprise a surface and a cross-sectional area. The area can have a regular shape or an irregular shape. The resistant to kinking can be due to the stiffer material, which has a higher modulus of elasticity or Young's modulus (E) and/or a higher moment of inertia compared to when the tube body is made from only a single softer or less stiff material.

The second portion 155 made from a second material stiffer than the first material of the first portion 152 can be called a spine or a catheter spine 155. As discussed above, the spine 155, i.e., the second portion 155 made from the second material with relatively stiffer property, can help to prevent or resist kinking of the catheter tube 150, which if occurs can block fluid flow through the catheter lumen 156. For example, after successful venipuncture, a kinked catheter tube can block IV solution to the patient. Thus, it is preferable to use a catheter tube that is resistant to kinking. The catheter tube of the present disclosure, with a first portion having a first material and a second portion having a second material, which is stiffer than the first material, is resistant to kinking.

In an example, as shown in FIGs. 3-5, the spine 155, or second material of the second portion, has a constant cross-sectional profile and extends longitudinally along an upper portion of the catheter body 151, on the same side as the tab 114 of the catheter hub 110 of FIG. 3 described above, or above a horizontal median plane of the catheter tube. That is, an exemplary embodiment of a catheter tube has a spine 155 formed as a narrow strip with a substantially constant cross-sectional profile extending between the proximal end of the catheter tube 150 and the distal end of the catheter tube 150. In some examples, the spine 155 forms part of the arcuate exterior of the catheter tube and part of the arcuate interior of the catheter tube 150. In some embodiments, the spine 155 forms a narrow strip that does not have a constant cross-sectional profile along the length of the catheter body 151. In other embodiments, the spine 155 has a variable cross-sectional profile along a length of the catheter body 151. For example, the distal portion of the catheter tube can be provided with a relatively narrow cross-sectional profile and the cross-sectional profile can increase in width as the length extends in the proximal direction. Still further, rather than having tapered sidewalls for the spine 155, the sidewalls can vary between straight, taper, undulating, tapering outwardly, etc.

The first portion 152 is joined together at the sides of the catheter spine 155 of the second portion to jointly form a seamless and smooth outer surface 158 of the catheter body 151, and an interior surface of the catheter body. This allows the catheter tube 150 to avoid snagging or shearing tissues when the catheter tube is inserted into the patient to access the vein and when feeding the catheter tube 150 to the desired location inside the vein. The first portion 152 and the catheter spine 155 may also be joined together to form a seamless catheter lumen 156 along the inner surface of the catheter tube. For example, the spine 155 can be co-extruded with the first portion 152 to form a seamless inner surface and outer surface.

As discussed above, in an embodiment, the spine 155 is oriented upwardly or along an upper portion similar to the top side or upper portion of the catheter body 151. Said differently, the spine 155 can make up the upper portion of the catheter body or catheter tube 151 and the first portion 152 can make up the remainder or at least the lower portion of the catheter body 151. As shown in FIG. 4, the cross-sectional profile of the spine or second portion 155 of the catheter body 151 can occupy about 25 to about 180 degrees of the arc of the catheter body 151 and the first portion 152 can occupy the remaining portion of the catheter body 151. The spine 155 may occupy more or less of the catheter body 151 depending on the outer diameter of the catheter body 151 and the desired overall stiffness of the catheter body 151. That is, the width of the cross-sectional profile and the shape of the spine 155 can determine the stiffness and, consequently, the desired length of the catheter body 151.

To increase the stiffness of a catheter tube 151, a material of the spine 155 can be chosen, for example, to be stiffer than a typical material used for the catheter tube. The relatively stiffer material selected for the spine can be used to form the entire catheter tube or only portions of the catheter tube while the remaining portions can be formed using typical or conventional catheter tube materials. In an example, the body of the catheter tube has at least two different materials used to form the length of the tube body, such as 50% or more of the length of the tube body. In an example, the material of the spine 155 should be harder than fluorinated ethylene propylene copolymer (FEP) material, which is typically used for a standard single material catheter body. Another exemplary material that is usable to form the first portion 152 is polyurethane (PUR). In some examples, the second portion can be made from FEP while the first portion is made from PUR.

In a particular example, barium sulfate (BaSO₄) can be used to form the spine. Thus, as a particular example, a catheter tube having a tube body with a lumen can be formed using FEP or PUR material with a BaSO₄ material and wherein the BaSO4 material is used to form a spine, or second portion 155, that runs lengthwise of the tube body and the FEP or PUR material forming the balance of the tube body, which can be referred to as the first portion 152. The first portion 152 can be made from a softer common catheter material, which can include polyurethane (PUR) or FEP. In an example, the BaSO₄ material is mixed with an effective amount of a polyether block amide (PEBA) or other compatible polymer materials to facilitate bonding with the first material, such as to facilitate bonding with the FEP or PUR material. Any suitable biocompatible material can be used for the second portion 155 as long as the material of the second portion used to form the spine has a greater stiffness property than the material used to form the first portion 152.

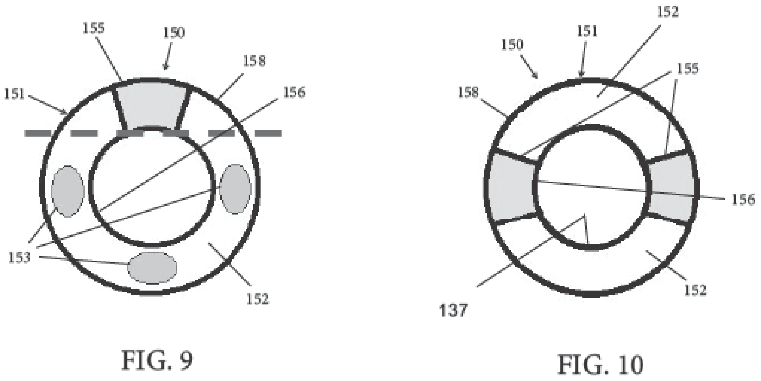

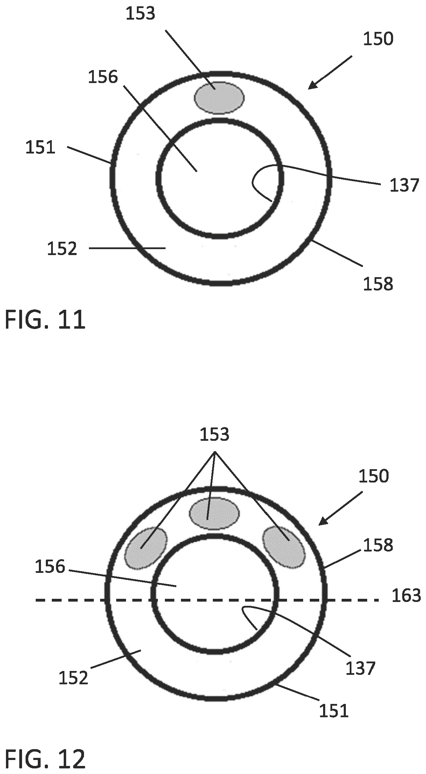

The catheter tube 150 can be manufactured by a co-extrusion manufacturing process. The second material used to form the spine 155 can be embedded within the inner and outer surfaces of the tube body 151 (such as shown in FIGs. 11 and 12) or can be co-extruded to form at least part of the exterior surface, the interior surface, or both the interior and exterior surfaces of the tube body (such as shown in FIGs. 9 and 10). In still other examples, the tube body 151 can have multiple spines or multiple spaced apart second portions that are formed within or encased by the first portion to form the tube body in accordance with aspects of the present catheter tubes. The multiple spines can be embedded or not embedded within the inner and outer surfaces of the tube body or there can be spines that are embedded and spines that are not embedded within the inner and outer surfaces of a tube body, as shown in FIG. 9.

The increased stiffness of the tube body 151 of a catheter tube when one or more strips of spines 155 are incorporated allows for a longer catheter tube 150 to be utilized. The catheter body 151 can be made stiffer, at least along the section or space occupied by the spine 155, to decrease the likelihood of bending or kinking. Because the lower portion of the catheter body 151 having first 151 and second 155 portions can be made from a softer material, the softer material to form the first portion 152 and the relatively stiffer or harder material to form the spine 155 of the second portion, the likelihood of injuries caused by contact made between the lower portion of the catheter body 151 and the interior wall tissues of a vein can be minimized.

With reference now to FIGs. 7 and 8 and particularly to the spine 155 being formed along the upper portion of the tube body 151 and the first portion 152 being formed with a more flexible material or less stiff material along the lower portion, the catheter tube 150 can be advanced into the vein after successful venipuncture with the needle removed from the catheter tube 150. During advancement of the catheter tube 150, the distal tip 149 of the tube body 151 may encounter the inside wall or venous wall 135 of the vein 130. A reaction force by the inside wall 135 of the vein counters the driving action of the catheter tube 150 is applied to the catheter tube 150 by the venous wall 135. The reaction force applied to the catheter tube 150 can cause the catheter tube 150 to deflect and the angle of deflection to increase, as shown in FIG. 8. More specifically, the reaction force would cause the lower portion of the catheter body 151 to bend upwardly, resulting in the lower portion being under tension and the upper portion of the catheter body 151, such as the second portion or the spine 151, experiencing at least some compression. However, because the catheter tube 150 is stiffer when one or more spines 155 are incorporated with the tube body in accordance with aspects of the present invention, the upward deflection is limited by the rigidity of the spine thereby allowing the distal tip of the catheter tube 150 to advance further into the vein without bending too far or too much upwardly, such as being bent substantially or completely upwardly, to contact the opposite side if the venous wall 135 and possibly kink.

For a typical catheter tube, if the deflection is too large, the catheter tube can form a tight bend or a kink, and as a result, reduce or prevent the flow of fluid through the catheter lumen. If the stiffness or Young's modulus of the catheter tube 150 is increased, such as by incorporating a spine 155 of the present disclosure, a larger force would be required to bend the catheter tube 150 and therefore reduce the likelihood of a kink formed in the catheter tube 150. The stiffness of the catheter tube 150 can be adjusted by changing the width or the shape of the cross-sectional profile of the spine 155, or decrease the number of spines used with the tube body. In still other examples, the relative stiffness between the second portion and the first portion can be selected based on selection of materials. The materials can be selected so that the material of the second portion compared to the material of the first portion can have a stiffness ratio of from about 1.05 to 1.8 of second material stiffness to first material stiffness. In still other examples, the stiffness ratio is selected to be greater than about 1.8 of second material stiffness to first material stiffness.

In one embodiment, the width of the cross-sectional profile of the spine 155 can be generally constant lengthwise from proximate the distal tip of the catheter body 151 and extending towards the proximal end of the catheter body 151. The spine 155 may or may not extend to the very proximal end of the catheter body 151. In another embodiment, the spine 155 may have a width or a cross-sectional profile that increases or varies from a point at the distal end of the catheter body or tube 151, such as a point just proximal of the tapered portion at the distal end or starting from the distal opening, and extending towards the proximal end of the catheter body or tube 151. In the embodiment with the increasing cross-sectional width, the stiffness of the tube body increases from the distal end of the tube body to the proximal end of the tube body 151. In still other examples, the distal most point of the spine 155 can originate proximal of the tapered portion 157, and up to several millimeters proximal of the tapered portion.

In addition to the location of the spine 155, the shape of the spine 155 can also contribute to the stiffness of the catheter tube 150. In one example, the spine 155 has an arcuate shaped structure, such as shown in FIGs. 4-5. The arcuate shaped structure of the spine 155 and the cylindrical shape of the catheter tube 150 as a whole allows the catheter tube 150 to extend in a straight configuration along a lengthwise direction. The stiffness of the spine and of the catheter tube can increase by increasing the width of the cross-sectional profile of the arcuate shaped spine 155, which has a larger width along the outer surface of the spine than the inside surface of the spine. As the width of the cross-sectional profile of the spine 155 increases, so does the height of the arcuate shaped spine 155, thereby dramatically increasing the moment of inertia of the spine 155.

As a comparison, when taking a ¼ section of the catheter tube and placing it on a flat surface compared to taking a ½ section of the catheter tube and placing it on a flat surface, the height of the ½ section is higher. Thus, by increasing the width of the cross-sectional profile of the spine, the height can also increase. The increase in moment of inertia also increases the stiffness of the spine 155. Simply stated, the stiffness of the catheter tube 150 can be adjusted by the shape of the spine 155. For example, the catheter tube can have a first stiffness when a spine having a cross-sectional profile of a first width and the catheter tube can have a second stiffness by changing the shape of the cross-sectional profile to a second width, which is larger than the first width. In yet another example, the stiffness can increase by changing the angles of the two sidewalls of the arcuate shaped spine 155. For example, looking at the sidewalls of the spine 155 of FIG. 4, the sidewalls can taper outward when extending from the exterior surface to the interior surface so that the interior arcuate surface is wider than the exterior arcuate surface.

FIG. 9 shows another embodiment of the catheter tube 150 having a catheter body 151, shown along an end cross-section. The catheter tube 150 in FIG. 9 is similar to the catheter tube 150 illustrated in FIGs. 2-6 except that the catheter tube 150 of FIG. 9 further includes one or more spines 153 that are embedded inside the wall thickness of the first portion 152. Three spines 153 are shown embedded with one, two, or more than three spines contemplated for use with the tube body. When more than one embedded spines 153 are incorporated, the spines 153 can be equally spaced or unequally spaced from one another. The spines 153 can be spaced away from the catheter lumen 156 and the outer surface 158 of the catheter body 151. As shown, the embedded spines 153 are enclosed or encased between the inner and outer surfaces of the tube body 151. Thus, an aspect of the invention can include a catheter tube with a tube body and wherein a spine or second section is provided with an exterior surface, an interior surface, or both exterior and interior surfaces that extend or flow from a first section of the tube body and wherein a second spine is enclosed or encased between the inner and outer surfaces of the first section of the tube body 151. Additional spines may be enclosed or encased by the first section of the tube body, which is made from a less tiff material than the material used to form the spine.

The shape of the spines 153 can be oval, circular, rectangular, or any other regular or irregular shape. The spines 153 can run lengthwise and extend between the proximal end and the distal end of the catheter body 151, including to the proximal-most and distal-most ends of the proximal and distal ends. The material of the spines 153 that are embedded can be the same material or a material that is softer than the material used to form the spine 155 that is not embedded. The spine 155 that is not embedded has a surface that is exposed along the exterior surface of the tube body, along the interior surface of the tube body, or both.

The material of the first portion 152 is softer than the materials of both the spines 153 that are embedded within the tube body 151, which are spaced from one another and have surfaces that are entirely within the interior and exterior surfaces of the tube body 151, and the spine 155 that is not embedded within the tube body 151, which has at least one surface that is exposed along the exterior surface of the tube body, along the interior surface of the tube body, or both. In an example, the spine 155 that is not embedded is made from BaSO₄, and the first portion 152 is made from polyurethane. Alternatively, the first portion 152 is made from silicone. In yet other examples, the first portion is made from polyethylene. In still other examples, the first portion 152 is made from a compound, such as from Teflon/PTFE.

The spines 153 that are embedded within the tube body 151 can be made from BaSO₄. If BaSO4 is used, the material for the spine can be blended with an affective amount of PEBA to facilitate bonding with the material of the first portion 152. BaSO₄ has properties that are sufficient for increasing the stiffness of the catheter tube and will provide X-ray visibility as well. Aspects of the present disclosure is directed to the use of the disclosed catheter tubes to limit or prevent kinking and to enable the fabrication of extended length tube bodies compared to tube bodies made from a single material or from a uniform blended composite.

In some examples, the relatively stiffer material used to form or make the spine 155 is PEEK or PROPELL^{TM}. In yet other examples, the second material used to make the second portion, or the spine, is bismuth subcarbonate (Bi₂O₂CO₃) or bismuth oxychloride (BiOCl).

When multiple strips of a second portion 155 are incorporated with a first portion 152 to form a tube body 151 of a catheter tube, the multiple strips of the second portion can be made from the same material or from different materials. For example, in a catheter tube embodiment with two embedded spines 153 and one non-embedded spine 155, BaSO₄ can be used to make the non-embedded spine and Bi₂O₂CO₃ can be used to make the embedded spines.

The spines can increase the overall stiffness of the catheter tube 150. The spines can be of the embedded type 153 or the non-embedded type 155 or both. In some examples, the tube body 151 can have multiple spine types, such as two or more embedded spines 153 and two or more non-embedded spines 155. When incorporated, the one or more spines should be positioned so that a stiffer region of the catheter tube is along the upper portion of the tube body. The spines 153 may also be located away from the distal tip and the tapered portion 154 of the catheter body 151 to ensure the distal tip of the catheter body 151 remains a softer first portion 152. The catheter tube can be made by a co-extrusion process.

In an example, the three embedded spines 153 of FIG. 9 can be made from a BaSO₄ material and can be used for X-ray visibility and optical transparency. The non-embedded spine 155 can be relatively larger, such as having a greater girth or width, than the embedded spines 153 to increase the stiffness of the tube body along the upper portion of the tube body. This arrangement has all the advantages of similar catheter bodies with a relatively stiffer upper portion described elsewhere herein.

FIG. 10 shows another embodiment of the catheter tube 150 having a catheter body 151, shown along an end cross-section. The catheter body 151 in FIG. 10 is similar to the catheter body 151 depicted in FIGs. 2-6 except that the catheter body 151 of FIG. 10 includes two non-embedded spines 155 spaced from each other by a first portion 152. A non-embedded spine, or a strip of material having a different stiffness property than the material used to form the tube body, is understood as one that has an exposed interior surface, exterior surface, or both exposed interior and exterior surfaces. It can be said that the embodiment of FIG. 10 has more spines than the embodiment of FIGs. 2-6.

As shown, the two spines 155 are not located at the upper portion of the catheter body 151 but along a horizontal median line passing through the tube body. The softer first material 152 is incorporated at both an upper portion of the catheter body 151 and at a lower portion of the catheter body 151. Therefore, the lower portion of the catheter body 151 that can contact the interior surface of the venous wall 135 during advancement of the catheter tube 150 would be the softer first portion 152 made of the first material to minimize potential injury or damage to the venous wall 135. In the embodiment of FIG. 10, the interior concave surfaces of the two spines 155 and the interior concave surfaces of the first portion 152 jointly form the catheter lumen 156 and the outer convex surfaces of the two spines 155 and the outer convex surfaces of the first portion 152 jointly form the exterior surface of the catheter tube. In one embodiment, the spines 155 can be made from a BaSO₄ material and the first portion 152 can be made from a polyurethane material. Optionally, an effective amount of PEBA can be incorporated with the BaSO₄ material to facilitate bonding.

The advantages of the catheter tube 150 with a spine of increased stiffness material compared to the material for forming the remaining portion of the tube body and located away from the lower portion of the catheter tube 150 include a stronger resistance to bending and kinking, while maintaining a soft lower portion that may contact the interior surface of the venous wall during advancement of the catheter tube 150 after venipuncture. The hardness and thus the stiffness of the spine 155 can be configured according to the need and application of the catheter device, assembly, or apparatus 100 or can be used for a standard length over-the-needle catheter, not just for extended length catheter tubes.

Bigger benefits can be derived when using the teachings of the present invention in connection with a relatively longer length catheter tube 150. Moreover, the increased stiffness of the catheter tube ensures patency, such as an unblocked lumen, thereby preventing infection or phlebitis, and reduction in pain. Another benefit of the increased stiffness is the capability of the catheter tube of the present invention to be advanced deep into a vein without a guidewire, although a guidewire can be used. The catheter tube 150 of the present invention is a kink resistant tube having at least two first and second portions made from two different materials with two different stiffness properties with a softer flexible material of the two specially located to minimize or prevent injury to the venous wall 135. For example, the softer flexible material can be located along a lower or bottom portion of the catheter body.

With reference now to FIG. 11, a catheter tube 150 having a tube body 151 provided in accordance with further aspects of the present invention is shown. The present catheter tube 150 is similar to other catheter tubes described elsewhere herein and comprises a first portion or section 152 made of a first material and a second portion or section 153 made of a second material. The tube body 151 has an exterior surface 158 and an interior surface 137 defining a lumen 156. In the present embodiment, the second portion or section 153 is a strip embedded into the first portion 152. The second portion 153, which can be called a spine, has surfaces that are entirely encased within the first portion 152. The first portion 152 may be made from PUR or PEBA material and the second portion 153 may be made from FEP material. In still other examples, the first portion 152 may be made from FEP, PUR, or PEBA material and the second portion 153 may be made from BaSO₄ material. As shown, the second portion 153 is located along the upper portion of the tube body 151, elevation wise.

With reference now to FIG. 12, a catheter tube 150 having a tube body 151 provided in accordance with still further aspects of the present invention is shown. The present catheter tube 150 is similar to other catheter tubes described elsewhere herein and comprises a first section or portion 152 made of a first material and a second section or portion 153 made of a second material. The tube body 151 has an exterior surface 158 and an interior surface 137 defining a lumen 156. In the present embodiment, the second portion 153 comprises three spaced apart strips embedded into the first portion 152. The three strips of the second portion 153, which can be called a spine, each having surfaces that are entirely encased within the first portion 152. The first portion 152 may be made from PUR or PEBA and the second portion 153, such as the three spines, may be made from FEP. In still other examples, the first portion 152 may be made from FEP, PUR, or PEBA and the second portion 153, such as the three spines, may be made from BaSO₄. As shown, the second portion 153 is located along the upper portion of the tube body 151, above a median line 163 passing through the center of the tube body.

Methods of making and of using over-the-needle catheter devices in which the catheter tubes have at least two different portions made from at least two different materials are within the scope of the invention.

Although limited embodiments of the intravenous catheter assemblies and their components including a catheter tube having a first portion and a second portion, the second portion can be one or more spines, have been specifically described and illustrated herein, many modifications and variations will be apparent to those skilled in the art. For example, the various intravenous catheter assemblies and catheter tubes with spines may incorporate other forms of spine characteristics, etc. Furthermore, it is understood and contemplated that features specifically discussed for one intravenous catheter assembly embodiment may be adopted for inclusion with another intravenous catheter assembly embodiment, provided the functions are compatible. For example, a catheter tube with a spine that is embedded may be used in another embodiment with a non-embedded configuration. Accordingly, it is to be understood that the intravenous catheter assemblies and their components constructed according to principles of the disclosed device, system, and method may be embodied other than as specifically described herein. The disclosure is also defined in the following claims.

## Claims

1. A catheter assembly (100) comprising:
a catheter hub (110) having a catheter tube (150) attached thereto;
a needle (101) with a needle tip (102) attached to a needle hub (103) and the needle (101) projecting through the catheter tube (150) with the needle tip (102) projecting distally of a distal opening (149) of the catheter tube (150);
the catheter tube (150) comprising a catheter body (151) having a wall with an exterior surface (158), an interior surface (137), a wall thickness between the exterior surface (158) and the interior surface (137), and a lumen (156) defined by the interior surface (137), the catheter body (151) comprising:
a first portion (152) of the wall thickness formed from a first material with a first stiffness property, the first portion (152) having an inner surface forming at least part of the interior surface (137) of the catheter body (151) and of the lumen (156) and an outer surface forming at least a part of the exterior surface (158) of the catheter body (151);
a second portion (155) of the wall thickness formed from a second material with a second stiffness property, the second portion (155) having an inner surface and an outer surface;
wherein the second stiffness property of the second material is greater than the first stiffness property of the first material;
wherein the second portion (155) is embedded within the wall thickness of the catheter tube (150) or not embedded within the wall thickness of the catheter tube (150); and
wherein when the second portion (155) is not embedded within the wall thickness of the catheter tube (150), (i) the inner surface of the second portion (155) forms another part of the interior surface (137) of the catheter body (151) and of the lumen (156), (ii) the outer surface of the second portion (155) forms another part of the exterior surface (158) of the catheter body (151), or (iii) the inner surface of the second portion (155) forms another part of the interior surface (137) of the catheter body (151) and of the lumen (156) and the outer surface of the second portion (155) forms another part of the exterior surface (158) of the catheter body (151).

2. The catheter assembly of claim 1, wherein the second portion (155) has a cross-sectional profile of a width that is generally constant along a length of the catheter tube (150).

3. The catheter assembly of claim 1, wherein a length of the catheter body (151) is between 1.4 cm to 6.4 cm or between 8 cm to 12 cm.

4. The catheter assembly of claim 1, wherein a distal end of the catheter tube (150) is tapered.

5. The catheter assembly of claim 1, wherein the first material comprises polyurethane (PUR) and has a stiffness property that is lower than the second stiffness property.

6. The catheter assembly of claim 1, wherein the first material comprises fluorinated ethylene propylene (FEP) and has a stiffness property that is lower than the second stiffness property.

7. The catheter assembly of claim 1, wherein the first material comprises polyether block amide (PEBA) and has a stiffness property that is lower than the second stiffness property.

8. The catheter assembly of claim 1, wherein the second material is barium sulfate (BaSO₄).

9. The catheter assembly of claim 1, wherein the second material is fluorinated ethylene propylene (FEP).

10. The catheter assembly of claim 1, wherein the first portion (152) and the second portion (155) extend from or proximate the distal opening (149) of the catheter body (151) towards a proximal end of the catheter body (151).

11. The catheter assembly of claim 1, wherein the catheter body (151) has three strips of spaced apart spines (155) each with a stiffness property greater than the first stiffness property.

12. The catheter assembly of claim 1, wherein the second portion (155) is a first spine and further comprising a second spine spaced from the first spine.

13. The catheter assembly of claim 1, further comprising a needle guard (104) having a surface configured to move distal of the needle tip (102) to cover the needle tip (102).

14. A method of forming a catheter assembly (100) comprising:
forming a catheter hub (110) having a catheter tube (150) attached thereto;
forming a needle hub (103) with a needle (101) having a needle tip (102) and projecting the needle (101) through the catheter tube (150) with the needle tip (102) projecting distally of a distal opening (149) of the catheter tube (150);
wherein the catheter tube (150) comprises a catheter body (151) having a wall with an exterior surface (158), an interior surface (137), a wall thickness between the exterior surface (158) and the interior surface (137), and a lumen (156) defined by the interior surface (137), the catheter body (151) comprising:
a first portion (152) of the wall thickness formed from a first material with a first stiffness property, the first portion (152) having an inner surface forming at least part of the interior surface (137) of the catheter body (151) and of the lumen (156) and an outer surface forming at least a part of the exterior surface (158) of the catheter body (151);
a second portion (155) of the wall thickness formed from a second material with a second stiffness property, the second portion (155) having an inner surface and an outer surface;
wherein the second stiffness property of the second material is greater than the first stiffness property of the first material;
wherein the second portion (155) is embedded within the wall thickness of the catheter tube (150) r not embedded within the wall thickness of the catheter tube (150); and
wherein when the second portion (155) is not embedded within the wall thickness of the catheter tube (150), (i) the inner surface of the second portion (155) forms another part of the interior surface (137) of the catheter body (151) and of the lumen (156), (ii) the outer surface of the second portion (155) forms another part of the exterior surface (158) of the catheter body (151), or (iii) the inner surface of the second portion (155) forms another part of the interior surface (137) of the catheter body (151) and of the lumen (156) and the outer surface of the second portion (155) forms another part of the exterior surface (158) of the catheter body (151).

15. A method of using a catheter assembly (100) with a catheter tube (150) having kinking resistant characteristics, said method comprising:
placing the catheter tube (150) into a vein (130), said catheter tube (150) attached to a catheter hub (110);
the catheter tube (150) comprising a catheter body (151) having a wall with an exterior surface (158), an interior surface (137), a wall thickness between the exterior surface (158) and the interior surface (137), and a lumen (156) defined by the interior surface (137), the catheter body (151) comprising:
a first portion (152) of the wall thickness formed from a first material with a first stiffness property, the first portion (152) having an inner surface forming at least part of the interior surface (137) of the catheter body (151) and of the lumen (156) and an outer surface forming at least a part of the exterior surface (158) of the catheter body (151);
a second portion (155) of the wall thickness formed from a second material with a second stiffness property, the second portion (155) having an inner surface and an outer surface;
wherein the second stiffness property of the second material is greater than the first stiffness property of the first material;
wherein the second portion (155) is embedded within the wall thickness of the catheter tube (150) or not embedded within the wall thickness of the catheter tube (150); and
wherein when the second portion (155) is not embedded within the wall thickness of the catheter tube (150), (i) the inner surface of the second portion (155) forms another part of the interior surface (137) of the catheter body (151) and of the lumen (156), (ii) the outer surface of the second portion (155) forms another part of the exterior surface (158) of the catheter body (151), or (iii) the inner surface of the second portion (155) forms another part of the interior surface (137) of the catheter body (151) and of the lumen (156) and the outer surface of the second portion (155) forms another part of the exterior surface (158) of the catheter body (151).

16. A catheter assembly (100) comprising:
a catheter hub (110) having a catheter tube (150) attached thereto;
a needle (101) with a needle tip (102) attached to a needle hub (103) and the needle (101) projecting through the catheter tube (150) with the needle tip (102) projecting distally of a distal opening (149) of the catheter tube (150);
the catheter tube (150) comprising a catheter body (151) having a wall with an exterior surface (158), an interior surface (137), a wall thickness between the exterior surface (158) and the interior surface (137), and a lumen (156) defined by the interior surface (137), the catheter body (151) comprising a first portion (152) and a second portion (155), and wherein the first portion (152) has a first stiffness property and the second portion (155) has a second stiffness property, and wherein the second stiffness property is greater than the first stiffness property.
